(19) Europäisches Patentamt
European Patent Office
Office européen des brevets

(11) Publication number: **0 010 617**
**B1**

(12) **EUROPEAN PATENT SPECIFICATION**

(45) Date of publication of patent specification: **05.08.81**

(21) Application number: **79103669.2**

(22) Date of filing: **27.09.79**

(51) Int. Cl.³: **C 07 D 401/04,**
C 07 D 403/04,
C 07 D 417/04,
C 07 D 413/00,
C 07 D 409/04,
A 61 K 31/40 //C07C109/04,
C07D213/55

(54) **Novel 2-substituted-3-heterocyclylindoles, their preparation and pharmaceutical compositions containing them.**

(30) Priority: **02.10.78 US 947979**

(43) Date of publication of application:
**14.05.80 Bulletin 80/10**

(45) Publication of the grant of the European patent:
**05.08.81 Bulletin 81/31**

(84) Designated Contracting States:
**AT BE CH DE FR GB IT NL SE**

(56) References cited:
**DE - A - 1 695 647**
**FR - A - 2 285 122**

(73) Proprietor: **SCHERING CORPORATION**
**2000 Galloping Hill Road**
**Kenilworth, New Jersey 07033 (US)**

(72) Inventor: **Steinman, Martin**
**46 Glendale Avenue**
**Livingston, New Jersey 07039 (US)**
Inventor: **Tahbaz, Pirouz**
**96 Oak Drive**
**Cedar Grove, New Jersey 07009 (US)**

(74) Representative: **Antony, Fritz, Dr. et al,**
**SCHERICO LTD. P.O. Box 601 Winkelriedstrasse 35**
**CH-6002 Lucerne (CH)**

**EP 0 010 617 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Novel 2-substituted-3-heterocyclylindoles, their preparation and pharmaceutical compositions containing them

This invention relates to novel 2-substituted-3-heterocyclylindoles, to their preparation, to their use as immuno-suppressants, and to pharmaceutical compositions containing them.

According to the invention we provide novel 2-substituted-3-heterocyclylindoles of the formula

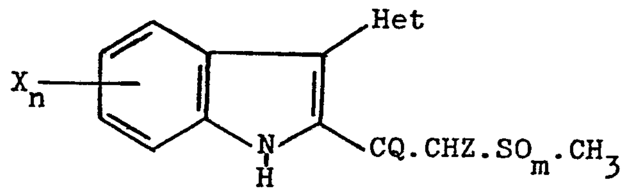

I

wherein X is a hydrogen or halogen atom,

*n* is 1, 2 or 3 (when X is a halogen atom),

Q is an oxygen atom or the group (H, OH),

Z is a hydrogen atom or, provided that Q is an oxygen atom, Z can also be the group OR, wherein R is a hydrogen atom or a lower alkyl or loweralkoxy-loweralkyl group, "lower" indicating groups with 1 to 6 carbon atoms,

*m* is 1 or 2 when Z is a hydrogen atom and *m* is 0 when Z is the group OR, and

Het is a heterocyclic radical selected from pyridyl, lower-alkyl-substituted-pyridyl, pyrimidinyl, pyridazinyl, pyrazinyl, pyrazolyl, imidazolyl, thiazolyl, isothiazolyl, thienyl, furanyl, pyrrolyl, oxazolyl and isoxazolyl.

The "Het" groups can be joint to the 3-position of the indole nucleus by any available ring atom, but are preferably joined through a carbon atom. Thus, for example, the pyridinyl groups can be 2-, 3- or 4-pyridinyl. A lower alkyl group substituting a pyridyl group is preferably methyl.

The term "halogen" comprises fluorine, chlorine, bromine and iodine. Halogen substituents in the fused benzene ring may be in any of the 4-, 5-, 6- or 7-positions; however, monosubstitution is preferably at the 5- or 6-position whereas polysubstitution is most preferably at the 5,6-positions but can also conveniently be at the 4,5- or 4,5,6-positions. $X_n$ thus preferably represents one or two halogen atoms, especially chlorine and/or bromine. In particular, $X_n$ can represent a 5-chlorine atom, a 5-chlorine atom together with a 4-bromine atom, or especially a 5-chlorine atom and a 6-bromine atom.

The 2-substituent of the indole nucleus is preferably a methylsulfonylacetyl or especially a methylsulfinylacetyl group; i.e. Q is an oxygen atom, Z is a hydrogen atom and *m* is 2 or especially 1. The 2-substituent can for example also be a methylthio-hydroxyacetyl, methylthio-loweralkoxy-acetyl (especially methylthio-methoxyacetyl), 2-methylsulfinyl-1-hydroxyethyl or 2-methylsulfonyl-1-hydroxyethyl group.

Preferred compounds according to the invention include

6-bromo-5-chloro-2-[(methylsulfinyl)acetyl]-3-(4-pyrimidinyl)indole (m.p. 206°C. (dec.)),
6-bromo-5-chloro-2-[(methylsulfinyl)acetyl]-3-(2-pyridyl) indole,
5-chloro-2-[(methylsulfinyl)acetyl]-3-(2-pyridyl) indole (m.p. 168—170°C.),
6-bromo-5-chloro-2-[(methylsulfinyl)acetyl]-3-(3-pyridyl)indole (m.p. 204.5°C), and
4-bromo-5-chloro-2-[(methylsulfinyl)acetyl]-3-(2-pyridyl)indole (m.p. 177—178°C.).

Other preferred compounds according to the invention include:

6-bromo-5-chloro-2-[(methylsulfinyl)acetyl]-3-(4-pyridyl)indole,
6-bromo-5-chloro-2-[(methylsulfinyl)acetyl]-3-(oxazolyl)indole,
6-bromo-5-chloro-2-[(methylsulfinyl)acetyl]-3-(isoxazolyl)indole,
6-bromo-5-chloro-2-[(methylsulfinyl)acetyl]-3-(pyrimidinyl)indole,
6-bromo-5-chloro-2-[(methylsulfinyl)acetyl]-3-(pyridazinyl)indole,
6-bromo-5-chloro-2-[(methylsulfinyl)acetyl]-3-(pyrazinyl)indole,
6-bromo-5-chloro-2-[(methylsulfinyl)acetyl]-3-(pyrazolyl)indole,
6-bromo-5-chloro-2-[(methylsulfinyl)acetyl]-3-(imidazolyl)indole,
6-bromo-5-chloro-2-[(methylsulfinyl)acetyl]-3-(thiazolyl)indole,
6-bromo-5-chloro-2-[(methylsulfinyl)acetyl]-3-(isothiazolyl)indole,
6-bromo-5-chloro-2-[(methylsulfinyl)acetyl]-3-(thienyl)indole,
6-bromo-5-chloro-2-[(methylsulfinyl)acetyl]-3-(furanyl)indole, and
6-bromo-5-chloro-2-[(methylsulfinyl)acetyl]-3-(pyrrolyl)indole.

A particularly preferred compound according to the invention is 6-bromo-5-chloro-2-[(methylsulfinyl)acetyl]-3-(2-pyridyl)indole (m.p. 198—199°C. (dec.)).

The invention further provides a process for the preparation of a compound of the formula I defined above, which comprises reacting an appropriate reactive derivative of an acid of the formula

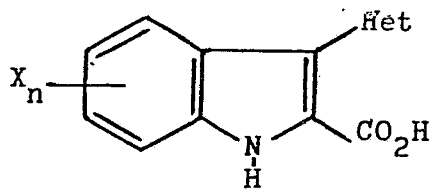

II

wherein Het, X and *n* are as defined above with an anion of the formula

$$^{\ominus}CH_2 . SO_m . CH_3 \quad III$$

wherein *m* is 1 or 2, in the presence of an anhydrous organic solvent and under an inert atmosphere, to yield an inert atmosphere, to yield a product of the formula

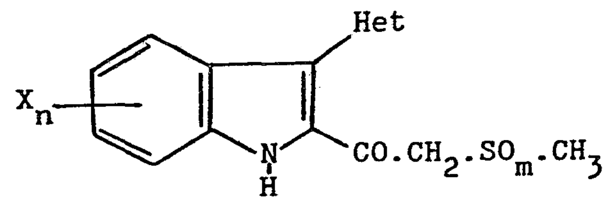

IA

wherein Hex, X, *m* and *n* are as defined above, wherafter, for the preparation of a compound of the formula I or IA wherein *m* is 2 when *m* in the product of the formula IA is 1, this product is oxidised,

and/or for the preparation of a compound of the formula I wherein Q is the group (H,OH), the product of the formula IA is reduced at the carbonyl group,

or, for the preparation of a compound of the formula I wherein Q is 0 and Z is the group OR wherein R is as defined above, a product of the formula IA wherein *m* is 1 is subjected to the action of the acid in the presence of a solvent comprising the compound ROH.

It will thus be understood that the products of the formula IA can either serve as final products of the formula I or be subjected to further steps to yield further products of the formula I.

The reactive derivative of the acid of the formula II is preferably an ester, especially a lower alkyl ester (the lower alkyl group having 1 to 6, preferably 1 to 4, carbon atoms), more especially the methyl or ethyl ester. The reactive derivative may also be the symmetrical anhydride of the acid or an N,N-disubstituted amide of the acid, e.g., an N,N-diloweralkylamide (wherein each lower alkyl group has 1 to 6, preferably 1 to 4 carbon atoms) or an N,N-pentamethylene amide.

The anion of the formula III can be prepared from dimethylsulfoxide or dimethylsulfone and a strong base, for example an alkali metal hydride, especially sodium hydride, an alkyl lithium, especially n-butyl-lithium, or potassium t-butoxide, by heating at a moderately elevated temperature, e.g., 60° to 75°C. for about two hours. If desired, an inert organic diluent or solvent, such as an aromatic hydrocarbon, especially benzene, may be present. Preparation of the anion of the formula III and its reaction with the derivative of the acid of the formula II should take place in an anhydrous reaction medium and under an inert atmosphere e.g. nitrogen.

The reaction between the anion of the formula III and the derivative of the acid of the formula II is preferably carried out by stirring the reactants at about room temperature until the reaction is complete (generally about 1 to 2 hours). The anion is preferably present in excess, e.g. about 3 equivalents in excess relative to the derivative of the acid of the formula II. After the reaction is complete, the reaction mixture is quenched with water and then acidified and the desired product is isolated for example by filtration and recrystallisation.

A so-obtained product of the formula IA wherein *m* is 1 can be oxidized to a corresponding product of the formula IA wherein *m* is 2. The oxidation is preferably effected by means of a peroxidic compound in an inert solvent, preferably hydrogen peroxide in acetic acid or an organic peracid in a halogenated organic solvent, e.g., a perbenzoic acid, especially *m*-chloroperbenzoic acid, in chloroform.

A product from those processes of the formula IA (wherein *m* is 1 or 2) can be preferentially reduced at the carbonyl group to yield a product of the formula I wherein Q is the group (H, OH), without reduction at the sulfinyl or sulfonyl group. This reduction is preferably effected by means of a borohydride, especially sodium borohydride, in a water-miscible inert organic solvent which may contain water, e.g. water-miscible ethers such as aqueous tetrahydrofuran or dioxan or lower alkanols, especially methanol or ethanol.

A product of the formula IA wherein *m* is 1 may be subjected to a Pummerer-type rearrangement in the presence of acid, preferably mineral acid, and a compound ROH (wherein R is as defined above) to yield a compound of the formula I wherein *m* is 0 and Z is OR (wherein R is as defined above). The mineral acid is conveniently hydrochloric acid. When this reaction is carried out in the presence of water, the product is a 2-[(methylthio)(hydroxy)acetyl]-3-heterocyclyl-indole (which may be called a 3-heterocyclyl-indole-2-glyoxal methyl hemimercaptal); when this reaction is carried out in the presence

of a lower alkanol (or loweralkoxy-loweralkanol), the product is a 2-[(methylthio)loweralkoxy- (or loweralkoxy-loweralkoxy)- acetyl]-3-heterocyclyl-indole.

The derivatives, e.g. the lower alkyl esters, of the 3-"Het'-indole-2-carboxylic acids of the formula II (wherein "Het" is a heterocyclic group as defined for formula I) either are known compounds or can be prepared by known methods. Thus an approximately $X_n$-substituted or unsubstituted phenylhydrazine hydrogen halide can be reacted with an appropriate lower alkyl β-"Het"-pyruvate, which is cyclized under conditions usual in the Fischer indole synthesis, i.e. using an acid catalyst e.g. a mineral acid such as sulfuric acid in acetic acid. This reaction often yields isomeric products, which can be separated by chromatography. For example, the cyclization of a 3,4-dihalophenylhyrazone of an alkyl β-(2-pyridyl)-pyruvate by heating with concentrated sulfuric acid in glacial acetic acid will produce a mixture of an alkyl 5,6-dihalo-3-(2-pyridyl)indole-2-carboxylate and an alkyl 4,5-dihalo-3-(2-pyridyl)-indole-2-carboxylate. The isomeric products are easily separated from this mixture by standard methods, e.g. by chromatography on silica gel in chloroform.

The required intermediates may be prepared by techniques well known to those of ordinary skill in the art. For example, ethyl 2-pyridylpyruvate-1-oxide is prepared according to Adams *et al.* (*J. Amer. Chem. Soc. 76,* 3168 (1954)), the succeeding ethyl 2-pyridylpyruvate according to S. Inaba *et. al.* (*Chem. Pharm. Bull., 20,* 1628 (1972)), the phenylhydrazines according to Bullock *et al.* (*J. Amer. Chem. Soc., 78,* 5854 (1956)), and their aniline precursors according to Suthers *et al.* (*J. Org. Chem., 27,* 447 (1962)).

The following preparations show how the starting materials for the process according to the invention can be obtained.

PREPARATION 1

3-Bromo-4-chlorophenylhydrazine hydrochloride

Treat 3-bromo-4-chloroaniline (41.3 g., 0.2 mole) in 90 ml. of 6N hydrochloric acid at −10°C. with 14 g. of sodium nitrite in 30 ml. of water. Add this solution with stirring to a cold (−10°C) solution of 55 g. of sodium bisulfite and 20 g. of sodium hydroxide in 200 ml. of water. After a red precipitate appears, allow the mixture to warm to room temperature. Add 200 ml. of concentrated hydrochloric acid and heat at 90—100°C. for 4 hours. Allow the mixture to cool overnight, collect the solid, wash it with 50 ml. of cold 3N hydrochloric acid and dry it. Recrystallization from ether-methanol yields the desired material, m.p. 208—210°C.

PREPARATION 2

3-Bromo-4-chlorophenylhydrazone of ethyl 2-pyridylpyruvate hydrochloride

Pass dry hydrogen chloride into 500 ml. of dry ethanol containing 54.4 g. of 3-bromo-4-chlorophenylhydrazine hydrochloride and 41.5 g. of ethyl 2-pyridylpyruvate until 50 g. of the dry acid is absorbed. With constant stirring reflux the resulting mixture for 2 hours; allow the mixture to cool overnight, collect the precipitate and wash it with cold ethanol, and dry it *in vacuo* to obtain the title compound, m.p. 297—208°C.

PREPARATION 3

Ethyl 6 - bromo - 5 - chloro - 3 - (2 - pyridyl)indole - 2 - carboxylate and Ethyl 4 - bromo - 5 - chloro-3 - (2 - pyridyl)indole - 2 - carboxylate

Add the 3-bromo-4-chlorophenylhydrazone of ethyl 2-pyridylpyruvate hydrochloride (86 g.) to 330 ml. of glacial acetic acid with stirring and warm to 70°C. Add 80 ml. of concentrated sulfuric acid dropwise over an hour, maintaining a temperature of 80—95°C. After a further 15 minutes, cool the mixture to 25°C. and pour it onto ice.

Adjust the pH to 8—9 with ammonia. Collect the precipitate, wash it with water and dry it.

Treat the precipitate with 200 ml. of methylene chloride and stir for one hour. Filter off the insoluble material which is mainly the 6-bromo-5-chloro isomer, wash it with methylene chloride and dry it. The filtrate contains both indoles.

Recrystallize the precipitate from benzene to yield the 6-bromo-5-chloro isomer, m.p. 173—175°. Further recrystallization from benzene (using charcoal) yields pure material (a single spot by thin layer chromatography); m.p. 179—180°C., analytical sample m.p. 181—182°C.

The methylene chloride filtrate is concentrated and cooled to yield light yellow crystals, m.p. 208—210°, of the 4-bromo-5-chloro isomer; analytical sample m.p. 209—210°C.

The following starting materials for the process according to the invention can be prepared analogously (or by other known methods):

ethyl 5-chloro-3-(2-pyridyl)indole-2-carboxylate,
ethyl 4,5,6-trichloro-3-(2-pyridyl)indole-2-carboxylate,
ethyl 5,6-dichloro-3-(2-pyridyl)indole-2-carboxylate,
ethyl 4,5-dichloro-3-(2-pyridyl)indole-2-carboxylate,
ethyl 6-chloro-3-(2-pyridyl)indole-2-carboxylate,
ethyl 4,6-dibromo-3-(2-pyridyl)indole-2-carboxylate,
ethyl 5-bromo-6-fluoro-3-(2-pyridyl)indole-2-carboxylate,
ethyl 5,6-difluoro-3-(2-pyridyl)indole-2-carboxylate,

ethyl 6-bromo-5-chloro-3-(3-pyridyl)indole-2-carboxylate,
ethyl 6-bromo-5-chloro-3-(4-pyridyl)indole-2-carboxylate,
ethyl 6-bromo-5-chloro-3-(2-oxazolyl)indole-2-carboxylate,
ethyl 6-bromo-5-chloro-3-(3-isoxazolyl)indole-2-carboxylate,
ethyl 6-bromo-5-chloro-3-(2-pyrimidinyl)indole-2-carboxylate,
ethyl 6-bromo-5-chloro-3-(3-pyridazinyl)indole-2-carboxylate,
ethyl 6-bromo-5-chloro-3-(2-pyrazinyl)indole-2-carboxylate,
ethyl 6-bromo-5-chloro-3-(3-pyrazolyl)indole-2-carboxylate,
ethyl 6-bromo-5-chloro-3-(2-imidazolyl)indole-2-carboxylate,
ethyl 6-bromo-5-chloro-3-(2-thiazolyl)indole-2-carboxylate,
ethyl 6-bromo-5-chloro-3-(3-isothiazolyl)indole-2-carboxylate,
ethyl 6-bromo-5-chloro-3-(2-thienyl)indole-2-carboxylate,
ethyl 6-bromo-5-chloro-3-(2-furanyl)indole-2-carboxylate,
ethyl 6-bromo-5-chloro-3-(2-pyrrolyl)indole-2-carboxylate, and
ethyl 6-bromo-5-chloro-3-(4-pyrimidinyl)indole-2-carboxylate.

The following Examples illustrate the preparation of compounds of the formula I:

## EXAMPLE I

6-Bromo-5-chloro-2-[(methylsulfinyl)acetyl]-3-pyridyl)indole

Add 6.2 g. of sodium hydride in mineral oil (50%) to 50 ml. of dry dimethylsulfoxide at 15—25°C. and then heat at 60—70°C. for two hours under nitrogen. Cool the mixture to 20°C. and add a suspension of 15.2 g. of ethyl 6-bromo-5-chloro-3-(2-pyridyl)indole-2-carboxylate in 50 ml. of dimethylsulfoxide at 15—25°C. Stir the mixture at ambient temperature for a further one hour, cool it to 15°C., add 10 ml. of water cautiously, and then add 190 ml. more. Filter, and add 10 ml. of acetic acid to the filtrate keeping the temperature below 20°C. Decant to obtain the gray solid product; stir this with ether, filter it off, wash it with ether and dry it to obtain the title compound, m.p. 198—199°C. (dec.).

The following compounds can be produced similarly from the 4-bromo-5-chloro isomer of Preparation 3 and from the list of compounds following Preparation 3:

4-bromo-5-chloro-2-[(methylsulfinyl)acetyl]-3-(2-pyridinyl)indole,
5-chloro-2-[(methylsulfinyl)acetyl]-3-(2-pyridyl)indole,
4,5,6-trichloro-2-[(methylsulfinyl)acetyl]-3-(2-pyridyl)indole,
5,6-dichloro-2-[(methylsulfinyl)acetyl]-3-(2-pyridyl)indole,
4,5-dichloro-2-[(methylsulfinyl)acetyl]-3-(2-pyridyl)indole,
6-chloro-2-[(methylsulfinyl)acetyl]-3-(2-pyridyl)indole,
4,6-dibromo-2-[(methylsulfinyl)acetyl]-3-(2-pyridyl)indole,
5-bromo-6-fluoro-2-[(methylsulfinyl)acetyl]-3-(2-pyridyl)indole,
5,6-difluoro-2-[(methylsulfinyl)acetyl]-3-(2-pyridyl)indole,
6-bromo-5-chloro-2-[(methylsulfinyl)acetyl]-3-(3-pyridyl)indole,
6-bromo-5-chloro-2-[(methylsulfinyl)acetyl]-3-(4-pyridyl)indole,
6-bromo-5-chloro-2-[(methylsulfinyl)acetyl]-3-(2-oxazolyl)indole,
6-bromo-5-chloro-2-[(methylsulfinyl)acetyl]-3-(3-isoxazolyl)indole,
6-bromo-5-chloro-2-[(methylsulfinyl)acetyl]-3-(2-pyrimidinyl)indole,
6-bromo-5-chloro-2-[(methylsulfinyl)acetyl]-3-(3-pyridazinyl)indole,
6-bromo-5-chloro-2-[(methylsulfinyl)acetyl]-3-(2-pyrazinyl)indole,
6-bromo-5-chloro-2-[(methylsulfinyl)acetyl]-3-(3-pyrazolyl)indole,
6-bromo-5-chloro-2-[(methylsulfinyl)acetyl]-3-(2-imidazolyl)indole,
6-bromo-5-chloro-2-[(methylsulfinyl)acetyl]-3-(2-thiazolyl)indole,
6-bromo-5-chloro-2-[(methylsulfinyl)acetyl]-3-(3-isothiazolyl)indole,
6-bromo-5-chloro-2-[(methylsulfinyl)acetyl]-3-(2-thienyl)indole,
6-bromo-5-chloro-2-[(methylsulfinyl)acetyl]-3-(2-furanyl)indole,
6-bromo-5-chloro-2-[(methylsulfinyl)acetyl]-3-(2-pyrrolyl)indole, and
6-bromo-5-chloro-2-[(methylsulfinyl)acetyl]-3-(4-pyrimidinyl)indole.

## EXAMPLE 2

6-Bromo-5-chloro-2-[(methylsulfonyl)acetyl]-3-(2-pyridyl)indole

Stir a mixture of 6.0 g. of 6-bromo-5-chloro-2-[(methylsulfinyl)acetyl]-3-(2-pyridyl)indole and 6.0 g. of *m*-chloroperbenzoic acid in 150 ml. of chloroform at room temperature for 2 hours. Concentrate the mixture to about 60 ml. *in vacuo*, filter off the product and wash it with 20 ml. of cold chloroform to obtain 6-bromo-5-chloro-2-[(methylsulfinyl)acetyl]-3-(2-pyridyl)indole.

By replacing the 6-bromo-5-chloro-2-[(methylsulfinyl)acetyl]-3-(2-pyridyl)indole with the compounds obtained by the method of Example 1 and by substantially following the method of this Example, the following compounds are produced:

4-bromo-5-chloro-2-[(methylsulfinyl)acetyl]-3-(2-pyridyl)indole,
5-chloro-2-[(methylsulfonyl)acetyl]-3-(2-pyridyl)indole,

4,5,6-trichloro-2-[(methylsulfonyl)acetyl]-3-(2-pyridyl)indole,
5,6-dichloro-2-[(methylsulfonyl)acetyl]-3-(2-pyridyl)indole,
4,5-dichloro-2-[(methylsulfonyl)acetyl]-3-(2-pyridyl)indole,
6-chloro-2-[(methylsulfonyl)acetyl]-3-(2-pyridyl)indole,
4,6-dibromo-2-[(methylsulfonyl)acetyl]-3-(2-pyridyl)indole,
5-bromo-6-fluoro-2-[(methylsulfonyl)acetyl]-3-(2-pyridyl)indole,
5,6-difluoro-2-[(methylsulfonyl)acetyl]-3-(2-pyridyl)indole,
6-bromo-5-chloro-2-[(methylsulfonyl)acetyl]-3-(3-pyridyl)indole,
6-bromo-5-chloro-2-[(methylsulfonyl)acetyl]-3-(4-pyridyl)indole,
6-bromo-5-chloro-2-[(methylsulfonyl)acetyl]-3-(2-oxazolyl)indole,
6-bromo-5-chloro-2-[(methylsulfonyl)acetyl]-3-(3-isoxazolyl)indole,
6-bromo-5-chloro-2-[(methylsulfonyl)acetyl]-3-(2-pyrimidinyl)indole,
6-bromo-5-chloro-2-[(methylsulfonyl)acetyl]-3-(3-pyridazinyl)indole,
6-bromo-5-chloro-2-[(methylsulfonyl)acetyl]-3-(2-pyrazinyl)indole,
6-bromo-5-chloro-2-[(methylsulfonyl)acetyl]-3-(3-pyrazolyl)indole,
6-bromo-5-chloro-2-[(methylsulfonyl)acetyl]-3-(2-imidazolyl)indole,
6-bromo-5-chloro-2-[(methylsulfonyl)acetyl]-3-(2-thiazolyl)indole,
6-bromo-5-chloro-2-[(methylsulfonyl)acetyl]-3-(3-isothiazolyl)indole,
6-bromo-5-chloro-2-[(methylsulfonyl)acetyl]-3-(2-thienyl)indole,
6-bromo-5-chloro-2-[(methylsulfonyl)acetyl]-3-(2-furanyl)indole,
6-bromo-5-chloro-2-[(methylsulfonyl)acetyl]-3-(2-pyrrolyl)indole, and
6-bromo-5-chloro-2-[(methylsulfonyl)acetyl]-3-(4-pyrimidinyl)indole.

## EXAMPLE 3

### 6-bromo-5-chloro-2-(1-hydroxy-2-methylsulfinyl)ethyl-3-(2-pyridyl indole

To 6-bromo-5-chloro-2-[(methylsulfinyl)acetyl]-3-(2-pyridyl)indole (2.5 g.) in 50 ml. of ethanol add 0.3 g. of sodium borohydride and, after a solution is obtained (about 10 minutes, stir the mixture for another 30 minutes. Slowly add 1 ml. of acetic acid and then add 100 ml. of water. Decant the liquid from the insoluble material, dissolve it in ethanol and add ether-hexane to obtain 6-bromo-5-chloro-2-[(1-hydroxy-2-methylsulfinyl)ethyl]-3-(2-pyridyl)indole.

Replacing the 6-bromo-5-chloro-2-[(methylsulfinyl)acetyl]-3-(2-pyridyl)indole with the compounds obtained by the method of Example 1 and substantially following the method of this Example provides the following compounds:

4-bromo-5-chloro-2-[(1-hydroxy-2-methylsulfinyl)ethyl]-3-(2-pyridyl)indole,
5-chloro-2-[(1-hydroxy-2-methylsulfinyl)ethyl]-3-(2-pyridyl)indole,
4,5,6-trichloro-2-[(1-hydroxy-2-methylsulfinyl)ethyl]-3-(2-pyridyl)indole.
5,6-dichloro-2-[(1-hydroxy-2-methylsulfinyl)ethyl]-3-(2-pyridyl)indole,
4,5-dichloro-2-[(1-hydroxy-2-methylsulfinyl)ethyl]-3-(2-pyridyl)indole,
6-chloro-2-[(1-hydroxy-2-methylsulfinyl)ethyl]-3-(2-pyridyl)indole,
4,6-dibromo-2-[(1-hydroxy-2-methylsulfinyl)ethyl]-3-(2-pyridyl)indole,
5-bromo-6-fluoro-2-[(1-hydroxy-2-methylsulfinyl)ethyl]-3-(2-pyridyl)indole,
5,6-difluoro-2-[(1-hydroxy-2-methylsulfinyl)ethyl]-3-(2-pyridyl)indole,
6-bromo-5-chloro-2-[(1-hydroxy-2-methylsulfinyl)ethyl]-3-(3-pyridyl)indole,
6-bromo-5-chloro-2-[(1-hydroxy-2-methylsulfinyl)ethyl]-3-(4-pyridyl)indole,
6-bromo-5-chloro-2-[(1-hydroxy-2-methylsulfinyl)ethyl]-3-(2-oxazolyl)indole,
6-bromo-5-chloro-2-[(1-hydroxy-2-methylsulfinyl)ethyl]-3-(3-isoxazolyl)indole,
6-bromo-5-chloro-2-[(1-hydroxy-2-methylsulfinyl)ethyl]-3-(2-pyrimidinyl)indole,
6-bromo-5-chloro-2-[(1-hydroxy-2-methylsulfinyl)ethyl]-3-(3-pyridazinyl)indole,
6-bromo-5-chloro-2-[(1-hydroxy-2-methylsulfinyl)ethyl]-3-(2-pyrazinyl)indole,
6-bromo-5-chloro-2-[(1-hydroxy-2-methylsulfinyl)ethyl]-3-(3-pyrazolyl)indole,
6-bromo-5-chloro-2-[(1-hydroxy-2-methylsulfinyl)ethyl]-3-(2-imidazolyl)indole,
6-bromo-5-chloro-2-[(1-hydroxy-2-methylsulfinyl)ethyl]-3-(2-thiazolyl)indole,
6-bromo-5-chloro-2-[(1-hydroxy-2-methylsulfinyl)ethyl]-3-(3-isothiazolyl)indole,
6-bromo-5-chloro-2-[(1-hydroxy-2-methylsulfinyl)ethyl]-3-(2-thienyl)indole,
6-bromo-5-chloro-2-[(1-hydroxy-2-methylsulfinyl)ethyl]-3-(2-furanyl)indole,
6-bromo-5-chloro-2-[(1-hydroxy-2-methylsulfinyl)ethyl]-3-(2-pyrrolyl)indole, and
6-bromo-5-chloro-2-[(1-hydroxy-2-methylsulfinyl)ethyl]-3-(4-pyrimidinyl)indole.

## EXAMPLE 4

### 6-Bromo-5-chloro-2-[(1-hydroxy-2-methylsulfonyl)ethyl]-3-(2-pyridyl)indole

To 6-bromo-5-chloro-2-[(methylsulfonyl)acetyl]-3-(2-pyridyl)indole (2.2 g.) in 75 ml. of ethanol add 0.175 g. of sodium borohydride. After a solution is obtained (about 10 minutes) stir the mixture for another 30 minutes, then slowly add 2 ml. of acetic acid. Treat with charcoal, filter, and add 100 ml. of water to the filtrate to obtain 6 - bromo - 5 - chloro - 2 - [(1 - hydroxy - 2 - methylsulfonyl)ethyl] - 3 - (2-pyridyl)indole. Similarly, replacing the 6 - bromo - 5 - chloro - 2 - [(2 - methylsulfonyl)acetyl] - 3 - (2-pyridyl)indole with the compounds obtained by the method of Example 2 and substantially following the method of this Example provides the following compounds:
4-bromo-5-chloro-2-[(1-hydroxy-2-methylsulfonyl)ethyl]-3-(2-pyridyl)indole,
5-chloro-2-[(1-hydroxy-2-methylsulfonyl)ethyl]-3-(2-pyridyl)indole,
4,5,6-trichloro-2-[(1-hydroxy-2-methylsulfonyl)ethyl]-3-(2-pyridyl)indole,
5,6-dichloro-2-[(1-hydroxy-2-methylsulfonyl)ethyl]-3-(2-pyridyl)indole,
4,5-dichloro-2-[(1-hydroxy-2-methylsulfonyl)ethyl]-3-(2-pyridyl)indole,
6-chloro-2-[(1-hydroxy-2-methylsulfonyl)ethyl]-3-(2-pyridyl)indole,
4,6-dibromo-2-[(1-hydroxy-2-methylsulfonyl)ethyl]-3-(2-pyridyl)indole,
5-bromo-6-fluoro-2-[(1-hydroxy-2-methylsulfonyl)ethyl]-3-(2-pyridyl)indole,
5,6-difluoro-2-[(1-hydroxy-2-methylsulfonyl)ethyl]-3-(2-pyridyl)indole,
6-bromo-5-chloro-2-[(1-hydroxy-2-methylsulfonyl)ethyl]-3-(3-pyridyl)indole,
6-bromo-5-chloro-2-[(1-hydroxy-2-methylsulfonyl)ethyl]-3-(4-pyridyl)indole,
6-bromo-5-chloro-2-[(1-hydroxy-2-methylsulfonyl)ethyl]-3-(2-oxazolyl)indole,
6-bromo-5-chloro-2-[(1-hydroxy-2-methylsulfonyl)ethyl]-3-(3-isoxazolyl)indole,
6-bromo-5-chloro-2-[(1-hydroxy-2-methylsulfonyl)ethyl]-3-(2-pyrimidinyl)indole,
6-bromo-5-chloro-2-](1-hydroxy-2-methylsulfonyl)ethyl]-3-(3-pyridazinyl)indole,
6-bromo-5-chloro-2-[(1-hydroxy-2-methylsulfonyl)ethyl]-3-(2-pyrazinyl)indole,
6-bromo-5-chloro-2-[(1-hydroxy-2-methylsulfonyl)ethyl]-3-(3-pyrazolyl)indole,
6-bromo-5-chloro-2-[(1-hydroxy-2-methylsulfonyl)ethyl]-3-(2-imidazolyl)indole,
6-bromo-5-chloro-2-[(1-hydroxy-2-methylsulfonyl)ethyl]-3-(2-thiazolyl)indole,
6-bromo-5-chloro-2-[(1-hydroxy-2-methylsulfonyl)ethyl]-3-(3-isothiazolyl)indole,
6-bromo-5-chloro-2-[(1-hydroxy-2-methylsulfonyl)ethyl]-3-(2-thienyl)indole,
6-bromo-5-chloro-2-[(1-hydroxy-2-methylsulfonyl)ethyl]-3-(2-furanyl)indole,
6-bromo-5-chloro-2-[(1-hydroxy-2-methylsulfonyl)ethyl]-3-(2-pyrrolyl)indole, and
6-bromo-5-chloro-2-[(1-hydroxy-2-methylsulfonyl)ethyl]-3-(4-pyrimidinyl)indole.

## EXAMPLE 5

### 6-Bromo-5-chloro-3-(2-pyridyl)indole-2-glyoxal methyl hemimercaptal

Slowly add 15 ml. of 6N hydrochloric acid to 6-bromo-5-chloro-2-[(methylsulfinyl)acetyl]-3-(2-pyridyl)indole (2 g.) in dimethylsulfoxide (75 ml.), stir the resulting mixture for 3 hours, quench with ice water, and collect and dry the solid to yield 6-bromo-5-chloro-3-(2-pyridyl)indole-2-glyoxal methyl hemimercaptal.

Replacing the 6-bromo-5-chloro-2-[(methylsulfinyl)acetyl]-3-(2-pyridyl)indole with the compounds obtained by the method of Example 1 and substantially following the method of this Example provides the following compounds:
4-bromo-5-chloro-3-(2-pyridyl)indole-2-glyoxal methyl hemimercaptal,
5-chloro-3-(2-pyridyl)indole-2-glyoxal methyl hemimercaptal,
4,5,6-trichloro-3-(2-pyridyl)indole-2-glyoxal methyl hemimercaptal,
5,6-dichloro-3-(2-pyridyl)indole-2-glyoxal methyl hemimercaptal,
4,5-dichloro-3-(2-pyridyl)indole-2-glyoxal methyl hemimercaptal,
6-chloro-3-(2-pyridyl)indole-2-glyoxal methyl hemimercaptal,
4,6-dibromo-3-(2-pyridyl)indole-2-glyoxal methyl hemimercaptal,
5-bromo-6-fluoro-3-(2-pyridyl)indole-2-glyoxal methyl hemimercaptal,
5,6-difluoro-3-(2-pyridyl)indole-2-glyoxal methyl hemimercaptal,
6-bromo-5-chloro-3-(3-pyridyl)indole-2-glyoxal methyl hemimercaptal,
6-bromo-5-chloro-3-(4-pyridyl)indole-2-glyoxal methyl hemimercaptal,
6-bromo-5-chloro-3-(2-oxazolyl)indole-2-glyoxal methyl hemimercaptal,
6-bromo-5-chloro-3-(3-isoxazolyl)indole-2-glyoxal methyl hemimercaptal,
6-bromo-5-chloro-3-(2-pyrimidinyl)indole-2-glyoxal methyl hemimercaptal,
6-bromo-5-chloro-3-(3-pyridazinyl)indole-2-glyoxal methyl hemimercaptal,
6-bromo-5-chloro-3-(2-pyrazinyl)indole-2-glyoxal methyl hemimercaptal,
6-bromo-5-chloro-3-(3-pyrazolyl)indole-2-glyoxal methyl hemimercaptal,
6-bromo-5-chloro-3-(2-imidazolyl)indole-2-glyoxal methyl hemimercaptal,
6-bromo-5-chloro-3-(2-thiazolyl)indole-2-glyoxal methyl hemimercaptal,
6-bromo-5-chloro-3-(3-isothiazolyl)indole-2-glyoxal methyl hemimercaptal,
6-bromo-5-chloro-3-(2-thienyl)indole-2-glyoxal methyl hemimercaptal,
6-bromo-5-chloro-3-(2-furanyl)indole-2-glyoxal methyl hemimercaptal,
6-bromo-5-chloro-3-(2-pyrrolyl)indole-2-glyoxal methyl hemimercaptal, and
6-bromo-5-chloro-3-(4-pyrimidinyl)indole-2-glyoxal methyl hemimercaptal.

## EXAMPLE 6

### 6-Bromo-5-chloro-2-[(methylthio)(methoxy)acetyl]-3-(2-pyridyl)indole

Add 2 ml. of concentrated hydrochloric acid to 6-bromo-5-chloro-2-[(methylsulfinyl)acetyl]-3-(2-pyridyl)indole (5.0 g.) in 50 ml. of methanol and 50 ml. of tetrahydrofuran and maintain the mixture at

60°C. for 2 hours. Cool, add water and collect and dry the resulting solid to obtain 6-bromo-5-chloro-2-[(methylthio)(methoxy)acetyl]-3-(2-pyridyl)indole.

Replacing the 6-bromo-5-chloro-2-[(methylsulfinyl)acetyl]-3-(2-pyridyl)indole with the compounds obtained by the method of Example 1 and substantially following the method of this Example provides the following compounds:
4-bromo-5-chloro-2-[(methylthio)(methoxy)acetyl]-3-(2-pyridyl)indole,
5-chloro-2-[(methylthio)(methoxy)acetyl]-3-(2-pyridyl)indole,
4,5,6-trichloro-2-[(methylthio)(methoxy)acetyl]-3-(2-pyridyl)indole,
5,6-dichloro-2-[(methylthio)(methoxy)acetyl]-3-(2-pyridyl)indole,
4,5-dichloro-2-[(methylthio)(methoxy)acetyl]-3-(2-pyridyl)indole,
6-chloro-2-[(methylthio)(methoxy)acetyl]-3-(2-pyridyl)indole,
4,6-dibromo-2-[(methylthio)(methoxy)acetyl]-3-(2-pyridyl)indole,
5-bromo-6-fluoro-2-[(methylthio)(methoxy)acetyl]-3-(2-pyridyl)indole,
5,6-difluoro-2-[(methylthio)(methoxy)acetyl]-3-(2-pyridyl)indole,
6-bromo-5-chloro-2-[(methylthio)(methoxy)acetyl]-3-(3-pyridyl)indole,
6-bromo-5-chloro-2-[(methylthio)(methoxy)acetyl]-3-(4-pyridyl)indole,
6-bromo-5-chloro-2-[(methylthio)(methoxy)acetyl]-3-(2-oxazolyl)indole,
6-bromo-5-chloro-2-[(methylthio)(methoxy)acetyl]-3-(3-isoxazolyl)indole,
6-bromo-5-chloro-2-[(methylthio)(methoxy)acetyl]-3-(2-pyrimidinyl)indole,
6-bromo-5-chloro-2-[(methylthio)(methoxy)acetyl]-3-(3-pyridazinyl)indole,
6-bromo-5-chloro-2-[(methylthio)(methoxy)acetyl]-3-(2-pyrazinyl)indole,
6-bromo-5-chloro-2-[(methylthio)(methoxy)acetyl]-3-(3-pyrazolyl)indole,
6-bromo-5-chloro-2-[(methylthio)(methoxy)acetyl]-3-(2-imidazolyl)indole,
6-bromo-5-chloro-2-[(methylthio)(methoxy)acetyl]-3-(2-thiazolyl)indole,
6-bromo-5-chloro-2-[(methylthio)(methoxy)acetyl]-3-(3-isothiazolyl)indole,
6-bromo-5-chloro-2-[(methylthio)(methoxy)acetyl]-3-(2-thienylindole,
6-bromo-5-chloro-2-[(methylthio)(methoxy)acetyl]-3-(2-furanyl)indole,
6-bromo-5-chloro-2-[(methylthio)(methoxy)acetyl]-3-(2-pyrrolyl)indole, and
6-bromo-5-chloro-2-[(methylthio)(methoxy)acetyl]-3-(4-pyrimidinyl)indole.

Similarly, replacing the methanol of this Example with equivalent quantities of straight and branched-chain lower alcohols having up to six carbon atoms provides the corresponding 2-[(methylthio) (lower alkoxy)acetyl]-3-(2-pyridyl)indoles and other 3-heterocyclylindoles.

Also included within the scope of the foregoing examples, particularly Example 2, are the 4-oxazolyl, 5-oxazolyl, 4-isoxazolyl, 5-isoxazolyl, 4-pyrimidinyl, 5-pyrimidinyl, 4-pyridazinyl, 4-pyrazolyl, 4-imidazolyl, 4-thiazolyl, 5-thiazolyl, 4-isothiazolyl, 5-isothiazolyl, 3-thienyl, 3-furanyl and 3-pyrrolyl isomeric forms thereof.

Since about 1960, immunosuppressive agents have found widespread clinical applications for treating diseases in which there is direct or indirect evidence for an immune etiology. Although treatment with corticosteroids has been successful in the clinical management of autoimmune diseases and in suppressing rejection phenomena associated with organ transplantation, patients are rendered highly susceptible to infection by this treatment. Indeed, there is a higher incidence of mortality from infections among these patients than from the diseases themselves.

The use of corticosteroids in such treatment can be reduced or even obviated by the use of azathioprine or cyclophosphamide, but it has none the less been very difficult to develop therapeutic regimens that yield clinical improvement in the absence of undesired side effects, notably bone marrow depression. Moreove, both these drugs have a slow onset of action so that therapeutically beneficial effects appear only after about three weeks' treatment.

We have now discovered that the novel 3-heterocyclyl-indoles of this invention have advantageous properties that indicate their usefulness in the treatment of autoimmune diseases and in suppressing rejection phenomena associated with organ transplantation.

Thus the compounds of formula I inhibit antibody immune reactions and cell-mediated immune reactions. The antibody immune reactions include the immune response to sheep erythrocytes and the immune response to trinitrophenylated liposaccharide in mice as assessed by the spleen assay of Jerne *et al.*, "Cell-bound Antibodies", Wistar Institute Press, 1963. Immune reactions classified as cell-mediated, delayed type hypersensitivities, include the late secondary migratory lesions in rats injected with Freund's adjuvant (in accordance with the techniques described in *British J. Pharmacology,* 21:127—136 and 24:632—640), skin transplant rejection in mice and rats, and mammary gland rejection (as described in "Transplantation of Cells and Tissues", Wistar Institute Press, 1961), contact and protein hypersensitivities in guinea pigs, rabbits and rats (Uhr, *Physiological Reviews,* 46: 359—419) and experimental allergic encephalomyelitis in rats.

From these tests and comparisons with known immunosuppressants, it may be determined that the compounds are effective in suppressing immune responses at about 0.5 to 50 mg./kg. mammalian body weight. Disease states against which the immunosuppressant activity of the compounds of this invention are useful include rheumatoid arthritis, ulcerative colitis, allergies, systemic lupus erythematosus, hemalytic anemia and Crohn's disease. In their use as immunosuppressants the

compounds have low toxicity and in particular are substantially non-cytotoxic at therapeutic doses.

For oral administration, the compounds of this invention may be combined with inert pharmaceutical carriers or excipients such as lactose, mannitol and starch. For parenteral injection, the compounds may be formulated with an inert, parenterally acceptable vehicle, such as water, saline or sesame oil. The formulations may be compounded according to methods well known to those skilled in the pharmaceutical art. Preferably the compounds are administered in 3—4 daily doses although the specific regimen will be dependent upon the severity and nature of the particular disease state.

The invention therefore provides pharmaceutical compositions comprising, as active ingredient, at least one compound of the formula I defined above in association with a suitable pharmaceutical carrier or excipient.

The compounds of the formula I can be administered in the form of dosage units, e.g., injectable dosage units in ampoules but in particular shaped dosage units such as tablets, capsules and suppositories. Dosage units conveniently contain from about 2 to about 300mg., preferably 10 to 50 mg., of active compound of the formula I.

*Example of a Pharmaceutical Preparation: Tablets*

| Ingredient | For 10, 000 tablets |
|---|---|
| Active ingredient of formula I | 250.0g. |
| Lactose | 1000.0 g. |
| Corn starch | 680.0 g. |
| Corn starch as 10% paste | 50.0 g. |
| Magnesium stearate | 20.0 g. |

Mix the active ingredient, lactose and 600 g. of the corn starch, and pass through a pulverizing mill if necessary. Granulate the so-obtained mixture with the starch paste, adding additional water if necessary to make a damp granulation. Pass the granulation through an impact mill to produce 8—12*mesh granules. Spread the granulation on trays and dry in a draft-oven at 35—40°C. Reduce the dried granulation to 16—24 mesh*size and blend it with the remaining 80.0 g. of corn starch and with the magnesium stearate until a uniform mixture is obtained. Compress to 200 mg. tablets containing 25 mg. of active ingredient.

In this Example, the active ingredient is preferably 6-bromo-5-chloro-2-[(methylsulfinyl)acetyl]-3-(2-pyridyl)indole, but may be any other compound of the formula I defined above, especially a compound named herein.

The following reports of tests illustrate the activity and low toxicity of a representative compound of the present invention:

*Test compound:*
6-Bromo-5-chloro-2-[(methylsulfinyl)acetyl]-3-(2-pyridyl)indole.

A. Humoral immunity in mice — plaque-forming cell responses

| | $ED_{50}$ |
|---|---|
| (i) to sheep erythrocytes (primary responses) | <1 mg./kg. |
| (ii) to sheep erythrocytes (secondary responses) | 0.42 mg./kg. |
| (iii) to trinitrophenylated liposaccharide | <0.1 mg./kg |

*These are U.S. standard sieves with openings (defined according to ASTM E—11—61) approximately as follows: 8—12 mesh: 2.36—1.68 mm.; 16—24 mesh: 1.19-about 0.73 mm.

B. Cellular immunity

| | $ED_{50}$ |
|---|---|
| (i) Graft *v.* host reaction in mice | c. 6 mg./kg. |
| (ii) Late secondary migration lesions in adjuvant rats | c 6 mg./kg. |
| (iii) Inhibition of paralysis in rats with experimental allergic encephalitis | <12.5 mg./kg. |

*Toxicity data*

1. No decrease in number of circulating white cells in rats at doses of 50 mg./kg. *p.o.* for 14 days.
2. No decrease in number of splenic leucocytes in mice at 640 mg./kg. *p.o.* for 8 days.
3. $LD_{50}$ in mice (dosed for 8 days) >1,000 mg./kg.
4. $LD_{50}$ in rats (dosed for 14 days) >100 mg./kg.

**Claims for the contracting states: BE, CH, DE, FR, GB, IT, NL and SE**

1. 2-Substituted-3-heterocyclyl-indoles of the formula

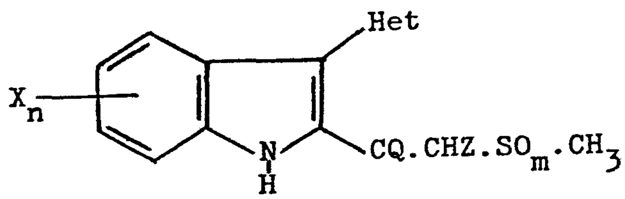

I

wherein X is a hydrogen or halogen atom,
*n* is 1, 2 or 3 (when X is a halogen atom),
Q is an oxygen atom or the group (H, OH),
Z is a hydrogen atom or, provided that Q is an oxygen atom, Z can also be the group OR, wherein R is a hydrogen atom or a lower alkyl or loweralkoxy-loweralkyl group, "lower" indicating groups with 1 to 6 carbon atoms,
*m* is 1 or 2 when Z is a hydrogen atom and *m* is 0 when Z is the group OR, and
Het is a heterocyclic radical selected from pyridyl, lower-alkyl-substituted-pyridyl, pyrimidinyl, pyridazinyl, pyrazinyl, pyrazolyl, imidazolyl, thiazolyl, isothiazolyl, thienyl, furanyl, pyrrolyl, oxazolyl and isoxazolyl.

2. Compounds as claimed in claim 1 wherein Het is a 3- or 4-pyridyl group or especially a 2-pyridyl group, or a 2-, 4- or 5-pyrimidinyl group, Q is an oxygen atom, Z is a hydrogen atom and *m* is 1, and $X_n$ represents two halogen atoms, preferably one chlorine and one bromine atom, especially a 5-chlorine and a 6-bromine atom.

3. A compound claimed in claim 1, namely 6-Bromo-5-chloro-2-[(methylsulfinyl)acetyl]-3-(2-pyridyl)indole.

4. Compounds claimed in claim 1, namely 5-Chloro-2-[(methylsulfinyl)acetyl]-3-(2-pyridyl)-indole, 4-Bromo-5-chloro-2-[methylsulfinyl)acetyl]-3-(2-pyridyl)indole, 6-Bromo-5-chloro-2-[(methyl-sulfinyl)acetyl]-3-(3-pyridyl)indole, and 6-Bromo-5-chloro-2-[(methylsulfinyl)acetyl]-3-(4-pyrimidinyl)-indole.

5. A process for the preparation of a compound claimed in claim 1, which comprises reacting a reactive derivative of an acid of the formula

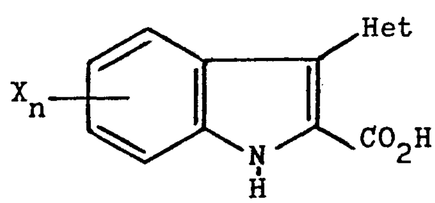

II

wherein Het, X and *n* are as defined in claim 1 with an anion of the formula

$$^{\ominus}CH_2 . SO_m . CH_3 \qquad III$$

wherein *m* is 1 or 2, in the presence of an anhydrous organic solvent and under an inert atmosphere, to yield an inert atmosphere, to yield a product of the formula

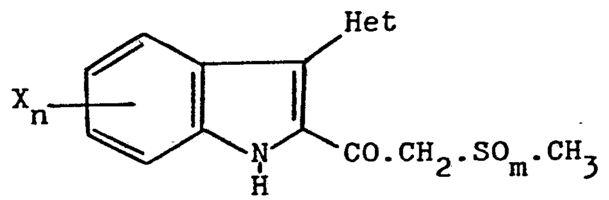

IA

wherein Hex, X, *m* and *n* are as defined in claim 1, whereafter, for the preparation of a compound of the formula I or IA wherein *m* is 2 when *m* in the product of the formula IA is 1, this product is oxidised, and/or for the preparation of a compound of the formula I wherein Q is the group (H,OH), the product of the formula IA is reduced at the carbonyl group,

or, for the preparation of a compound of the formula I wherein Q is O and Z is the group OR wherein R is as defined above, a product of the formula IA wherein *m* is 1 is subjected to the action of the acid in the presence of a solvent comprising the compound ROH.

6. A process as claimed in claim 5, wherein the reactive derivative is a lower alkyl ester, preferably an ethyl ester, and/or wherein the anion is present in excess, preferably in about 3 equivalents excess.

7. Pharmaceutical compositions containing as active ingredient at least one compound as claimed in any of claims 1 to 4 together with a pharmaceutical carrier or excipient.

8. Compositions as claimed in claim 7 in the form of dosage units, such as tablets, capsules or suppositories, especially dosage units containing from 2 to 300 mg. of active ingredient, preferably from 10 to 50 mg. of active ingredient.

9. Compositions as claimed in claim 7 in the form of injectable compositions.

10. Compositions as claimed in any of claims 7 to 9 wherein the active ingredient is 6-Bromo-5-chloro-2-[(methylsulfinyl)acetyl]-3-(2-pyridyl)indole.

**Patentansprüche für die Vertragsstaaten: BE, CH, DE, FR, GB, IT, NL, SE**

1. 2-Substituierte 3-Heterocyclylindole der Formel

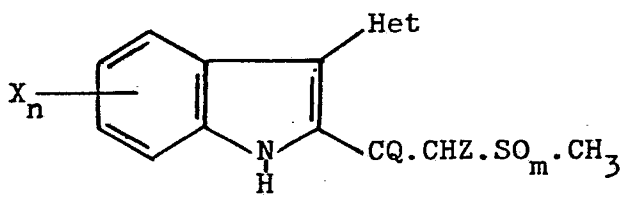

I

in der X ein Wasserstoff- oder Halogenatom ist,

n für 1, 2 oder 3 steht (wenn X ein Halogenatom ist),

Q ein Sauerstoffatom oder die Gruppe (H, OH) ist,

Z ein Wasserstoffatom ist oder, vorausgesetzt, daß Q ein Sauerstoffatom ist, auch die Gruppe OR sein kann, worin R ein Wasserstoffatom oder ein neiderer Alkylrest oder ein Niederalkoxy-niederalkylrest ist, wobei "nieder" Gruppen mit 1 bis 6 C-Atomen anzeigt,

m für 1 oder 2 steht, wenn Z ein Wasserstoffatom ist, und für O steht, wenn Z die Gruppe OR ist, und

Het ein aus Pyridyl, mit niederem Alkyl substituiertem Pyridyl, Pyrimidinyl, Pyridazinyl, Pyrazinyl, Pyrazolyl, Imidazolyl, Thiazolyl, Isothiazolyl, Thienyl, Furanyl, Pyrrolyl, Oxazolyl und Isoxazolyl ausgewählter heterocyclischer Rest ist.

2. Verbindungen nach Anspruch 1, worin Het eine 3- oder 4-Pyridylgruppe oder insbesondere eine 2-Pyridylgruppe oder ein 2-, 4- oder 5-Pyrimidinylgruppe,

Q ein Sauerstoffatom, Z ein Wasserstoffatom ist und

m für 1 steht und $X_n$ zwei Halogenatome, vorzugsweise ein Chloratom und ein Bromatom, insbesondere ein 5-Chloratom und ein 6-Bromatom darstellt.

3. Eine Verbindung nach Anspruch 1, nämlich 6-Brom-5-chlor-2-[methylsulfinyl)acetyl]-3-(2-pyridyl)indol.

4. Verbindungen nach Anspruch 1, nämlich 5-Chlor-2-[(methylsulfinyl)acetyl]-3-(2-pyridyl)indol, 4-Brom-5-chlor-2-[(methylsulfinyl)acetyl]-3-(2-pyridyl)indol,

6-Brom-5-chlor-2-[(methylsulfinyl)acetyl]-3-(3-pyridyl)indol und
6-Brom-5-chlor-2-[(methylsulfinyl)acetyl]-3-(4-pyrimidinyl)indol.

5. Verfahren zur Herstellung einer in Anspruch 1 beanspruchten Verbindung, dadurch gekennzeichnet, daß man ein reaktionsfähiges Derivat einer Säuer der Formel

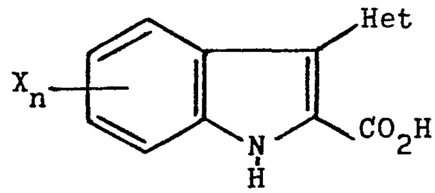

II

in der Het, X und n die in Anspruch 1 genannten Bedeutungen haben, mit einem Anion der Formel

$$^{\ominus}CH_1.SO_m.CH_3$$

III

in der für 1 oder 2 steht,
in Gegenwart eines wasserfreien organischen Lösungsmittels und unter einer inerten Atmosphäre umsetzt und hierbei ein Produkt der Formel

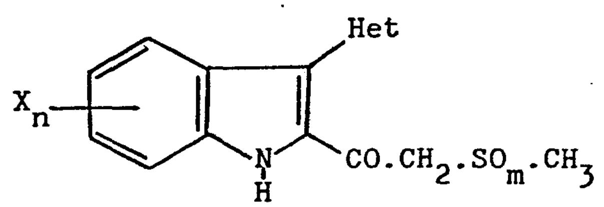

IA

bildet, in der Het, X, m und n die in Anspruch 1 genannten Bedeutungen haben, worauf man zur Herstellung einer Verbindung der Formel I oder IA, in der m für 2 steht, wenn m im Produkt der Formel IA für 1 steht, dieses Produkt oxidiert und/oder zur Herstellung einer Verbindung der Formel I, in der Q die Gruppe (H, OH) ist, das Produkt der Formel IA an der Carbonylgruppe reduziert oder zur Herstellung einer Verbindung der Formel I, in der Q für O und Z für die Gruppe OR steht, in der R die in Anspruch 1 genannte Bedeutung hat, ein Produkt der Formel IA, in der m für 1 steht, der Einwirkung einer Säure in Gegenwart eines die Verbindung ROH enthaltenden Lösungsmittels unterwirft.

6. Verfahren nach Anspruch 5, worin das reaktionsfähige Derivat ein niederer Alkylester, vorzugsweise ein Äthylester ist und/oder worin das Anion im Überschuß, vorzugsweise im Überschuß von etwa 3 Äquivalenten vorhanden ist.

7. Arzneimittelzubereitung, enthaltend als Wirkstoff wenigstens eine Verbindung nach irgendeinem der Ansprüche 1 bis 4 zusammen mit einem pharmazeutischen Träger oder Hilfsstoff.

8. Zubereitungen nach Anspruch 7 in Form von Dosierungseinheiten, z.B. Tabletten, Kapseln oder Suppositorien, indbesondere Dosierungseinheiten, die 2 bis 300 mg des Wirkstoffs, vorzugsweise 10 bis 50 mg des Wirkstoffs enthalten.

9. Zubereitungen nach Anspruch 7 in form von injizierbaren Zubereitungen.

10. Zubereitungen nach Ansprüchen 7 bis 9, worin der Wirkstoff 6-Brom-5-chlor-2-[(methylsulfinyl)acetyl]-3-(2-pyridyl)indol ist.

**Revendications pour les états contractants: BE, CH, DE, FR, GB, IT, NL, SE.**

1. 2-substitué-3-hétérocyclyl-indoles, caractérisés en ce qu'ils ont la formule:

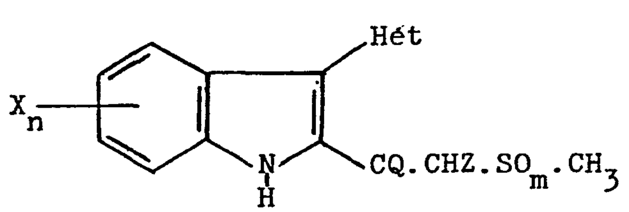

I

dans laquelle X représente un atome d'hydrogène ou d'halogène,
*n* est égal à 1, 2 ou 3 (lorsque X représente un atome d'halogène),
Q représente un atome d'oxygène ou le groupe (H, OH),
Z représente un atome d'hydrogène ou, pourvu que Q représente un atome d'oxygène, Z peut

également représenter le groupe OR, dans lequel R est un atome d'hydrogène ou un groupe alcoyle inférieur ou alcoxy inférieur-alcoxy inférieur, "inférieur" indiquant des groupes ayant de 1 à 6 atomes de carbone,

*m* est égal à 1 ou 2 lorsque Z représente un atome d'hydrogène et *m* représente O lorsque Z représente le groupe OR, et

Hét représente un radical hétérocyclique choisi parmi pyridyl, alcoyle inférieur-substitué pyridyle, pyrimidinyle, pyridazinyle, pyrazinyle, pyrazolyle, imidazolyle, thiazolyle, isothiazolyle, thiényle, furanyle, pyrrolyle, oxazolyle et isoxazolyle.

2. Composés selon la revendication 1, caractérisés en ce que Hét. représente un groupe 3- ou 4-pyridyle ou spécialement un groupe 2-pyridyle, ou un groupe 2-, 4- ou 5-pyrimidinyle,
Q représente un atome d'hydrogène, Z représente un atome d'hydrogène et
*m* est égal à 1, et $X_n$ représente deux atomes d'halogène, de préference un atome de chlore et un atome de brome, spécialement un atome 5-chlore et un atome 6-brome.

3. Composé selon la revendication 1, caractérisé en ce qu'il s'agit du 6-bromo-5-chloro-2-[(méthylsulfinyl)acétyl]-3-(2-pyridyle)indole.

4. Composé selon la revendication 1, caractérisé en ce qu'il s'agit de 5-chloro-2-[(méthylsulfinyl)acétyl]-3-(2-pyridyl)indole,
4-bromo-5-chloro-2-[(méthylsulfinyl)acétyl]-3-(2-pyridyl)indole,
6-bromo-5-chloro-2-[(méthylsulfinyl)acétyl]-3-(3-pyridyl)indole, et
6-bromo-5-chloro-2-[(méthylsulfinyl)acétyl]-3-(4-pyrimidinyl)indole.

5. Procédé de préparation d'un composé selon la revendication 1, caractérisé en ce qu'il comprend la réaction d'un dérivé réactif d'un acide de la formule:

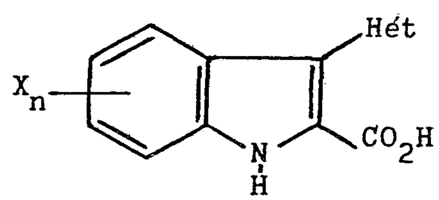

II

dans laquelle Hét., X et *n* sont tels que définis à la revendication 1, avec un anion de la formule:

$$^{\ominus}CH_2.SO_mCH_3 \qquad III$$

dans laquelle *m* est égal à 1 ou 2, en présence d'un solvant organique anhydre et sous atmosphère inerte, pour obtenir un produit de la formule:

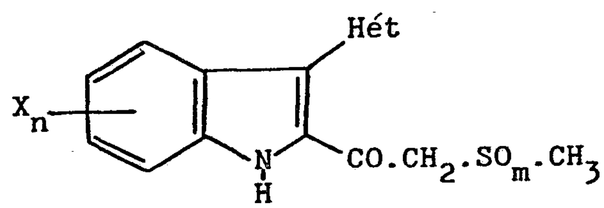

IA

dans laquelle Hét., X, *m* et *n* sont tels que définis à la revendication 1, après quoi, pour la préparation d'un composé de la formule I ou IA dans laquelle *m* est égal à 2 lorsque *m* dans le produit de la formule IA est égal à 1, ce produit est oxydé, et/ou pour la préparation d'un composé de la formule I dans laquelle Q représente le groupe (H, OH), le produit de la formule IA est réduit au groupe carbonyle, ou, pour la préparation d'un composé de la formule I dans laquelle Q représente O et Z représente le groupe OR dans lequel R est tel que défini à la revendication 1, un produit de la formule IA dans laquelle *m* est égal à 1 est soumis à l'action d'acide en présence d'un solvant comprenant le composé ROH.

6. Procédé selon la revendication 5, caractérisé en ce que le dérivé réactif est un ester d'alcoyle inférieur, de préférence un ester d'éthyle, et/ou en ce que l'anion est présent en excès, de préférence à environ 3 équivalents en excès.

7. Compositions pharmaceutiques, caractérisées en ce qu'elles contiennent comme ingrédient actif au moins un composé tel que revendiqué dans l'une quelconque des revendications 1 à 4 avec un support ou excipient pharmaceutique.

8. Compositions selon la revendication 7, caractérisées en ce qu'elles se trouvent sous la forme d'unités de dosage, telles que comprimés, capsules ou suppositoires, spécialement des unités de dosage contenant de 2 à 300 mg d'ingredient actif, de préférence de 10 à 50 mg d'ingrédient actif.

9. Compositions selon la revendication 7, caractérisées en ce qu'elles se trouvent sous la forme de compositions injectables.

10. Compositions selon l'une quelconque des revendications 7 à 9, caractérisées en ce que l'ingrédient actif est le 6-bromo-5-chloro-2-[(méthylsulfinyl)acétyl]-3-(2-pyridyl)indole.

**Claims for the contracting state: AT.**

1. A process for the preparation of 2-substituted-3-heterocyclyl-indoles of the formula

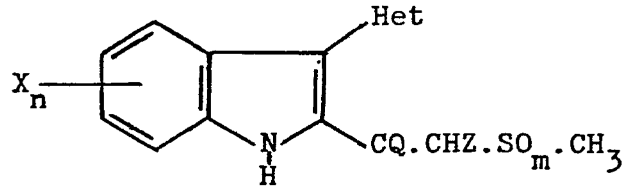

I

wherein X is a hydrogen or halogen atom,
*n* is 1, 2 or 3 (when X is a halogen atom),
Q is an oxygen atom or the group (H, OH),
Z is a hydrogen atom or, provided that Q is an oxygen atom, Z can also be the group OR, wherein R is a hydrogen atom or a lower alkyl or loweralkoxy-loweralkyl group, "lower" indicating groups with 1 to 6 carbon atoms,
*m* is 1 or 2 when Z is a hydrogen atom and *m* is 0 when Z is the group OR, and
Het is a heterocyclic radical selected from pyridyl, lower-alkyl-substituted-pyridyl, pyrimidinyl, pyridazinyl, pyrazinyl, pyrazolyl, imidazolyl, thiazolyl, isothiazolyl, thienyl, furanyl, pyrrolyl, oxazolyl and isoxazolyl, characterised in that a reactive derivative of an acid of the formula

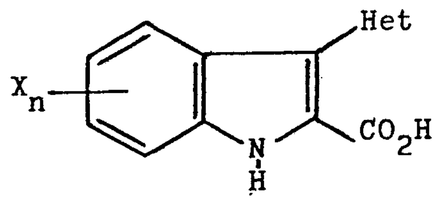

II

wherein Het, X and *n* are as defined above is reacted with an anion of the formula

$$^{\ominus}CH_2 . SO_m . CH_3 \qquad III$$

wherein *m* is 1 or 2, in the presence of an anhydrous organic solvent and under an inert atmosphere, to yield a product of the formula

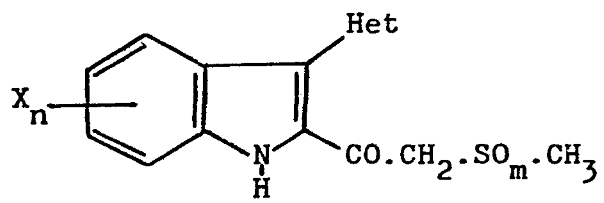

IA

wherein Hex, X, *m* and *n* are as defined above, wherafter, for the preparation of a compound of the formula I or IA wherein *m* is 2 when *m* in the product of the formula IA is 1, this product is oxidised, and/or for the preparation of a compound of the formula I wherein Q is the group (H,OH), the product of the formula IA is reduced at the carbonyl group,
or, for the preparation of a compound of the formula I wherein Q is O and Z is the group OR wherein R is as defined above, a product of the formula IA wherein *m* is 1 is subjected to the action of the acid in the presence of a solvent comprising the compound ROH.

2. A process as claimed in claim 1, characterised in that the reactive derivative is a lower akyl ester, preferably an ethyl ester.

3. A process as claimed in claim 1 or claim 2, characterised in that the anion is present in excess, preferably in about 3 equivalents excess.

4. A process as claimed in any of claims 1 to 3, characterised in that a product of the formula IA wherein *m* is 1 is oxidised to a product of the formula I wherein *m* is 2 with *m*-chloroperbenzoic acid in a halogenated organic solvent.

5. A process as claimed in any of claims 1 to 4, characterised in that the product of the formula IA is reduced with a borohydride in an inert water-miscible organic solvent to yield a compound of the formula I wherein Q is the group (H,OH).

6. A process as claimed in any of claims 1 to 3, characterised in that a product of the formula IA in which *m* is 1 is rearranged in the presence of a mineral acid and water or a lower alkanol.

7. A process as claimed in any of claims 1 to 3 for the preparation of 6-bromo-5-chloro-2-[(methylsulfinyl)acetyl]-3-(2-pyridyl)indole, characterised in that a reactive derivative, preferably a lower alkyl ester, of 6-bromo-5-chloro-3-(2-pyridyl)-indole-2-carboxylic acid is reacted with the anion of dimethyl sulfoxide.

8. A process as claimed in any of claims 1 to 3 for the preparation of
5-chloro-2-[(methylsulfinyl)acetyl]-3-(2-pyridyl)indole,
4-bromo-5-chloro-2-[(methylsulfinyl)acetyl]-3-(2-pyridyl)indole,
6-bromo-5-chloro-2-[(methylsulfinyl)acetyl]-3-(3-pyridyl)indole, or
6-bromo-5-chloro-2-[(methylsulfinyl)acetyl]-3-(4-pyrimidinyl)indole,
characterised in that a reactive derivative, preferably a lower alkyl ester, of the corresponding acid selected from 5-chloro-3-(2pyridyl)indole-2-carboxylic acid,
4-bromo-5-chloro-3-(2-pyridyl)indole-2-carboxylic acid,
6-bromo-5-chloro-3-(3-pyridyl)indole-2-carboxylic acid, and
6-bromo-5-chloro-3-(4-pyrimidinyl)indole-2-carboxylic acid, is reacted with the anion of dimethylsulfoxide.

9. A process for the preparation of a therapeutic composition comprising as active ingredient a compound of the formula I defined in claim 1, characterised in that the said active ingredient is brought into a form suitable for therapeutic administration.

10. A process as claimed in claim 9, characterised in that a compound of the formula I prepared by a process as claimed in any of claims 1 to 8 is admixed with a pharmaceutical carrier or excipient.

**Patentansprüche für den Vertragsstaat: AT.**

1. Verfahren zur Herstellung von 2-substituierten 3-Heterocyclyl-indolen der Formel

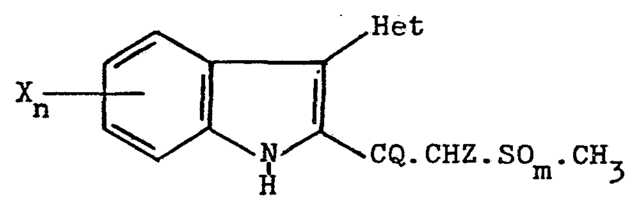

I

in der X ein Wasserstoff- oder Halogenatom ist,

n für 1, 2 oder 3 steht (wenn X ein Halogenatom ist),

Q ein Sauerstoffatom oder ein Gruppe (H, OH) ist,

Z ein Wasserstoffatom ist oder, vorausgesetzt, daß Q ein Sauerstoffatom ist, auch die Gruppe OR sein kann, worin R ein Wasserstoffatom oder ein niederer Alkylrest oder ein Niederalkoxy-niederalkylrest ist, wobei "nieder" Gruppen mit 1 bis 6 C-Atomen anzeigt,

m für 1 oder 2 steht, wenn Z ein Wasserstoffatom ist, und für O steht, wenn Z die Gruppe OR ist, und

Het ein aus Pyridyl, mit niederem Alkyl substituiertem Pyridyl, Pyrimidinyl, Pyridazinyl, Pyrazinyl, Pyrazolyl, Imidazolyl, Thiazolyl, Isothiazolyl, Thienyl, Furanyl, Pyrrolyl, Oxazolyl und Isoxazolyl ausgewählter heterocyclischer Rest ist, dadurch gekennzeichnet, daß man ein reaktionsfähiges Derivat einer Säure der Formel

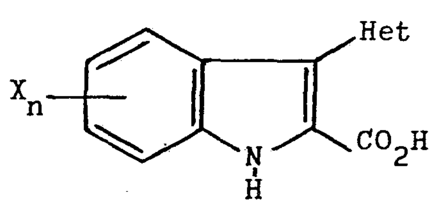

II

in der Het, X und n die vorstehend genannten Bedeutungen haben, mit einem Anion der Formel

$$^{\ominus}CH_2 \cdot SO_m \cdot CH_3 \qquad III$$

in der m für 1 oder 2 steht, in Gegenwart eines wasserfreien organischen Lösungsmittels und unter einer inerten Atmosphäre umsetzt und hierbei ein Produkt der Formel

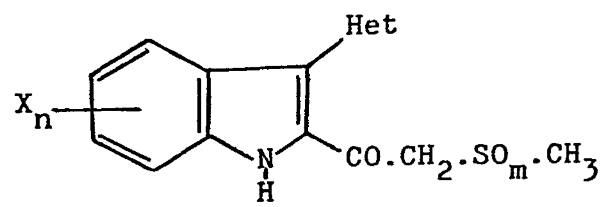

IA

in der Het, X, m und n die vorstehend genannten Bedeutungen haben, bildet worauf man zur Herstellung einer Verbindung der Formel I oder IA, in der m für 2 steht, wenn m im Produkt der Formel IA für 1 steht, dieses Produkt oxidiert und/oder zur Herstellung einer Verbindung der Formel I, in der Q die Gruppe (H, OH) ist, das Produkt der Formel IA an der Carbonylgruppe reduziert oder zur Herstellung einer Verbindung der Formel I, in der Q für O und Z für die Gruppe OR steht, in der R die vorstehend genannte Bedeutung hat, ein Produkt der Formel IA, in der m für 1 steht, der Einwirkung einer Säure in Gegenwart eines die Verbindung ROH enthalten-den Lösungsmittels unterwirft.

2. Verfahren nach Anspruch 1, dadurch gekennzeichnet, daß das reaktionsfähige Derivat ein niederer Alkylester, vorzugsweise ein Äthylester, ist.

3. Verfahren nach Anspruch 1 oder Anspruch 2, dadurch gekennzeichnet, daß das Anion im Überschuß, vorzugsweise in einem Überschuß von etwa 3 Äquivalenten, vorhanden ist.

4. Verfahren nach einem der Ansprüche 1 bis 3, dadurch gekennzeichnet, daß ein Produkt der Formel IA, in der m für 1 steht, zu einem Produkt der Formel I in m für 2 steht, mit m-Chlorperbenzoesäure in einem halogenierten organischen Lösungsmittel oxidiert wird.

5. Verfahren nach einem der Ansprüche 1 bis 4, dadurch gekennzeichnet, daß das Produkt der Formel IA mit einem Borhydrid in einem inerten, mit Wasser mischbaren organischen Lösungsmittel reduziert wird, wobei eine Verbindung der Formel I, in der Q die Gruppe (H, OH) ist, gebildet wird.

6. Verfahren nach einem der Ansprüche 1 bis 3, dadurch gekennzeichnet, daß ein Produkt der Formel IA, in der m für 1 steht, in Gegenwart einer Mineralsäure und Wasser oder eines niederen Alkonols umgelagert wird.

7. Verfahren nach einem der Ansprüche 1 bis 3 zur Herstellung von 6-Brom-5-chlor-2-[(methylsulfinyl)acetyl]-3-(2-pyridyl)indol, dadurch gekennzeichnet, daß ein reaktionsfähiges Derivat, vorzugsweise ein niederer Alkylester, von 6-Brom-5-chlor-3-(2-pyridyl)-indol-2-carbonsäure mit dem Anion von Dimethylsulfoxid umgesetzt wird.

8. Verfahren nach einem der Ansprüche 1 bis 3 zur Herstellung von
5-Chlor-2-[(methylsulfinyl)acetyl]-3-(2-pyridyl)indol,
4-Brom-5-chlor-2-{[(methylsulfinyl)acetyl]-3-(2-pyridyl-indol,
6-Brom-5-chlor-2-[(methylsulfinyl)acetyl]-3-(3-pyridyl)indol oder
6-Brom-5-chlor-2-[(methylsulfinyl)acetyl]-3-(4-pyrimidinyl)indol, dadurch gekennzeichnet, daß ein reaktionsfähiges Derivat, vorzugsweise ein niederer Alkylester, der entsprechenden, aus
5-Chlor-3-(pyridyl)indol-2-carbonsäure,
4-Brom-5-chlor-3-(2-pyridyl)indol-2-carbonsäure,
6-Brom-5-chlor-3-(3-pyridyl)indol-2-carbonsäure und
6-Brom-5-chloro-3-(4-pyrimidinyl)indol-2-carbonsäure ausgewählten Säure mit dem Anion von Dimethylsulfoxid umgesetzt wird.

9. Verfahren zur Herstellung einer Arzneimittelzubereitung, die als aktives Ingrediens eine Verbindung der in Anspruch 1 definierten Formel I enthält, dadurch gekennzeichnet, daß das aktive Ingrediens in eine für die therapeutische Verabreichung geeignete Form gebracht wird.

10. Verfahren nach Anspruch 9, dadurch gekennzeichnet, daß eine nach einem der in irgendeinem der Ansprüche 1 bis 8 beanspruchten Verfahren hergestellte Verbindung der Formel I mit einem pharmazeutischen Träger oder Hilfsstoff gemischt wird.

**Revendications pour l'état contractant: AT.**

1. Procédé de préparation de 2-substitué-3-hétérocyclyl-indoles de formule:

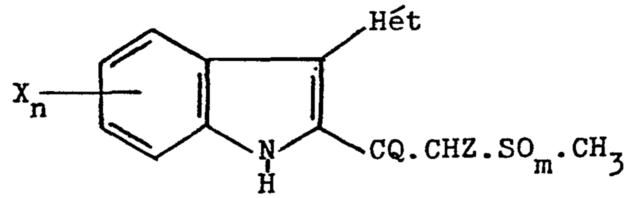

I

dans laquelle X représente un atome d'hydrogène ou d'halogène,
n est égal à 1, 2 ou 3 (lorsque X représente un atome d'halogène),
Q représente un atome d'oxygène ou le groupe (H, OH),
Z représente un atome d'hydrogène ou, lorsque Q représente un atome d'oxygène, Z peut

également représenter le groupe OR, dans lequel R représente un atome d'hydrogène ou un groupe alcoyle inférieur ou alcoxy inférieur-alcoyle inférieur, le terme "inférieur" indiquant des groupes ayant de 1 à 6 atomes de carbone.

m est égal à 1 ou 2 lorsque Z représente un atome d'hydrogène et m représente O lorsque Z représente le groupe OR, et

Hét. représente un radical hétérocyclique choisi parmi les radicaux pyridyle, pyridyle substitué par un groupe alcoyle inférieur, pyramidinyle, pyridazinyle, pyrazinyle, pyrazolyle, imidazolyle, thiazolyle, isothiazolyle, thiényle, furanyle, pyrrolyle, oxazolyle et isoxazolyle,

caractérisé en ce qu'un dérivé réactif d'un acide de la formule:

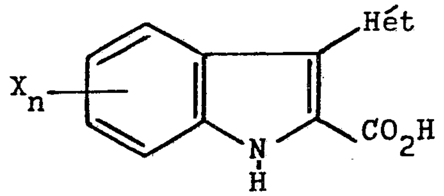

II

dans laquelle Hét., X et n sont tels que précédemment définis est mis à réagir avec un anion de la formule:

$$^{\ominus}CH_2.SO_m.CH_3 \qquad III$$

dans laquelle m est égal à 1 ou 2, en présence d'un solvant organique anhydre et sous atmosphère inerte, pour obtenir un produit de la formule:

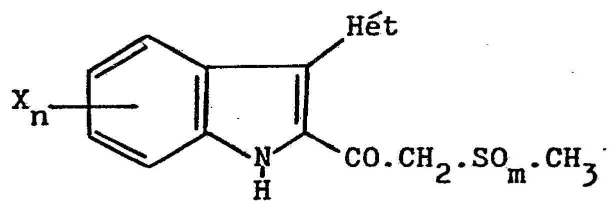

IA

dans laquelle Hét., X, m et n sont tels que précédemment définis, après quoi, pour la préparation d'un composé de la formule I ou IA dans laquelle m est égal à 2 lorsque m dans le produit de la formule IA est égal à 1, ce produit est oxydé, et/ou pour la préparation d'un composé de la formule I dans laquelle Q représente le groupe (H, OH), le produit de la formule IA est réduit au groupe carbonyle, ou, pour la préparation d'un composé de la formule I dans laquelle Q représente O et Z représente le groupe OR dans lequel R est tel que précédemment défini, un produit de la formule IA dans laquelle m est égal à 1 est soumis à l'action d'un acide en présence d'un solvant comprenant le composé ROH.

2. Procédé selon la revendication 1, caractérisé en ce que le dérivé réactif est un ester d'alcoyle inférieur, de préférence un ester d'éthyle.

3. Procédé selon la revendication 1 ou 2, caractérisé en ce que l'anion précité est présent en excès, de préférence en environ 3 équivalents d'excès.

4. Procédé selon l'une quelconque des revendications 1 à 3, caractérisé en ce qu'un produit de la formule IA dans laquelle m est égal à 1 est oxydé en un produit de la formule I dans laquelle m est égal à 2 avec l'acide m-chloroperbenzoique dans un solvant organique halogéné.

5. Procédé selon l'une quelconque des revendications 1 à 4, caractérisé en ce que le produit de la formule IA est réduit par un borohydrure dans un solvant organique miscible à l'eau inerte pour obtenir un composé de la formule I dans laquelle Q représente le groupe (H, OH).

6. Procédé selon l'une quelconque des revendications 1 à 3, caractérisé en ce qu'un produit de la formule IA dans laquelle m est egal à 1 est réarrangé en présence d'un acide minéral et d'eau ou d'un alcanol inférieur.

7. Procédé selon l'une quelconque des revendications 1 à 3, pour la préparation de 6-bromo-5-chloro-2-[(méthylsulfinyl)acétyl]-3-(2-pyridyl)indole, caractérisé en ce qu'un dérivé réactif, de préférence un ester d'alcoyle inférieur, d'acide 6-bromo-5-chloro-3-(2-pyridyl)-indole-2-carboxylique est mis a réagir avec l'anion du diméthyle sulfoxyde.

8. Procédé selon l'une quelconque des revendications 1 à 3, pour la préparation de:
5-chloro-2-[(méthylsulfinyl)acétyl]-3-(2pyridyl)indole,
4-bromo-5-chloro-2-[(méthylsulfinyl)acétyl]-3-(2-pyridyl)indole,
6-bromo-5-chloro-2-[(méthylsulfinyl)acétyl]-3-(3-pyridyl)indole, ou
6-bromo-5-chloro-2-[(méthylsulfinyl)acétyl]-3-(4-pyrimidinyl)indole,
caractérisé en ce qu'un dérivé réactif, de préférence un ester d'alcoyle inférieur, de l'acide correspondant choisi parmi:

l'acide 5-chlor-3-(2-pyridyl)indole -2-carboxylique,
l-acide 4-bromo-5-chloro-3-(2-pyridyl)indole-2-carboxylique,
l'acide 6-bromo-5-chloro-3-(3-pyridyl)indole-2-carboxylique, et
l'acide 6-bromo-5-chloro-3-(4-pyrimidinyl)indole-2-carboxylique, est mis à réagir avec l'anion du diméthylsulfoxyde.

9. Procédé de préparation d'une composition thérapeutique comprenant comme ingrédient actif un composé de la formule I définie à la revendication 1, caractérisé en ce que ledit ingrédient actif est mis sous une forme appropriée pour l'administration thérapeutique.

10. Procédé selon la revendication 9, caractérisé en ce qu'un composé de la formule I préparé par un procédé selon l'une quelconque des revendication 1 à 8 est mélangé avec un support ou excipient pharmaceutique.